Europäisches Patentamt

European Patent Office

Office européen des brevets

(19)

(11) Veröffentlichungsnummer: **0 250 369 B1**

(12) **EUROPÄISCHE PATENTSCHRIFT**

(45) Veröffentlichungstag der Patentschrift: **22.05.91**

(51) Int. Cl.⁵: **A61M 1/28**

(21) Anmeldenummer: **87810345.6**

(22) Anmeldetag: **16.06.87**

(54) **Dreiwegverteiler zum Flüssigkeitsaustausch.**

(30) Priorität: **20.06.86 CH 2506/86**

(43) Veröffentlichungstag der Anmeldung:
**23.12.87 Patentblatt 87/52**

(45) Bekanntmachung des Hinweises auf die
Patenterteilung:
**22.05.91 Patentblatt 91/21**

(84) Benannte Vertragsstaaten:
**BE DE ES FR GB IT NL SE**

(56) Entgegenhaltungen:
**US-A- 4 306 976**

(73) Patentinhaber: **Contempo Products, P. Herrli**
**Alpenstrasse 15a**
**CH-2502 Biel(CH)**

(72) Erfinder: **Herrli, Peter**
**Alpenstrasse 15a**
**CH-2502 Biel(CH)**

(74) Vertreter: **Schweizer, Hans et al**
**Bovard AG Patentanwälte VSP Optingen-**
**strasse 16**
**CH-3000 Bern 25(CH)**

## Beschreibung

Die Erfindung geht aus von einem Dreiwegverteiler zum Flüssigkeitsaustausch gemäss dem Oberbegriff des Patentanspruches 1 und einer Einrichtung zum Ausführen einer peritonealen Heimdialyse.

Ein solcher Dreiwegverteiler wird insbesondere in einem System zum Ausführen eines ambulanten dialytischen Entzuges von durch geschädigte Nieren eines Patienten ausgeschiedenen Stoffwechselprodukten eingesetzt. In einem solchem Fall wird aus der Bauchhöhle des Patienten verbrauchtes Dialysat abgeführt und nachher in dieselbe frisches Dialysat eingeführt.

Eine kontinuierliche ambulante peritoneale Dialyse (CAPD) kann von einem nierengeschädigten Patienten selber ohne Unterbrechung während 24 Stunden pro Tag und sieben Tage in der Woche ausgeführt werden. Die Heimdialyse wird in Zyklen von etwa sechs Stunden ausgeführt. Das in die Bauchhöhle des Patienten aus einem Beutel einzuführende frische Dialysat hat ein Volumen von meistens zwei Litern. Das verbrauchte Dialysat wird in einen leeren Beutel abgeführt. Durch die Heimdialyse werden von den geschädigten Nieren des Patienten ausgeschiedene Wechselstoffsubstanzen aus dem Körper des Patienten entfernt, wobei er sich während den Wechselzyklen seiner üblichen täglichen Tätigkeit widmen kann.

Zur Ausführung der peritonealen Heimdialyse wurde anfänglich ein Beutelsystem verwendet, das vom Patienten stets getragen werden musste.

Um die Kontaminationsgefahr weitgehend zu vermeiden und die Unbehaglichkeit des Tragens der Beutel am Körper des Patienten zu beseitigen, wird in der PCT-Anmeldung CH 84/0020 für die Zuführung des frischen Dialysats aus einem Beutel und für die Abführung des verbrauchten Dialysats in einen zweiten Beutel ein Y-Verbindungsstück verwendet. Nach dem Abführen des verbrauchten Dialysats wird der zum Beutel mit verbrauchtem Dialysat führende Schlauch durch eine Klemme abgeklemmt. Zum Durchspülen des Y-Verbindungsstückes wird eine Desinfektionsflüssigkeit verwendet, die sich in dem mit dem Beutel mit frischem Dialysat verbundenen Schlauch befindet, oder das Y-Verbindungsstück wird mit frischem Dialysat durchgespült.

Die Bedienungstechnik ist hier ziemlich kompliziert. Der Patient muss das Brechstück am Beutel mit frischem Dialysat abbrechen und den Rollschieber zur Regulierung der Auslaufgeschwindigkeit des frischen Dialysats einstellen. Darüber hinaus müssen auch Schlauchklemmen verwendet werden. Auch hier besteht aber noch immer die Gefahr einer Kontamination durch Bakterienkeime, weil sich die Bakterienkeime im offenen Y-Verbindungsstück und insbesondere auch im Arm der Leitung für den Durchgang des Frischdialysats verbreiten können. Das Y-Verbindungsstück mit den beiden Beuteln ist wegwerfbar, so dass bei einer neuen Dialyse ein neues System mit zwei Beuteln und einem Y-Verbindungsstück an den Anschlussschlauch angekoppelt werden muss.

Die oben beschriebene Vorrichtung erlaubt zwar die Abkoppelung des Beutelsystems, so dass der Patient zwischen den Dialysatwechselvorgängen das gesamte Beutelsystem nicht mehr mit sich herumtragen muss. Sie ist aber andererseits in ihrer Handhabung derart kompliziert, dass dafür nur eine gewisse Patientenauswahl in Frage kommt. Die Fehlmanipulation mit dieser Vorrichtung stellt für den Patienten stets ein erhebliches Risiko exogener Peritonitis dar.

Im US-Patent 4,306 976 ist ebenfalls ein Verfahren und eine Einrichtung zur ambulanten Peritonealdialyse beschrieben. Die Verbindung zwischen dem Beutelsystem und dem Katheter geschieht dabei über ein eine hohle Nadel aufweisendes Kupplungsstück. Mit dieser Nadel werden nacheinander zwei Membranen des Kupplungsstückes durchstochen. Zur Verringerung der Kontaminationsgefahr ist der Zwischenraum zwischen diesen Membranen mit einer sterilisierenden Lösung gefüllt. Dieser Vorgang sowie die Betätigung von den Durchfluss steuernden Mitteln schliesst auch hier Fehlmanipulationen und Infektionsgefahren nicht mit Sicherheit aus.

Der im Patentanspruch 1 angegebenen Erfindung liegt die Aufgabe zugrunde, einen Dreiwegverteiler zum Flüssigkeitsaustausch zu schaffen, bei welchem die Folgen einer eventuellen Kontamination der ihn durchlaufenden Flüssigkeiten ausgeschlossen werden. Desweiteren soll die Bedienung eines solchen Dreiwegverteilers einfach, komfortabel und leicht erlernbar sein. Die Fehlmanipulation mit dem Dreiwegverteiler soll durch geeignete Sicherungsmassnahmen und Ausschliessen zusätzlicher Bedienungsmittel vermieden werden.

Diese Aufgabe wird erfindungsgemäss durch die Merkmale des kennzeichnenden Teiles des Patentanspruches 1 gelöst. Der Dreiwegverteiler wird erfindungsgemäss in einem System zum Ausführen einer peritonealen Heimdialyse bei Patienten mit geschädigten Nieren verwendet.

Die Erfindung wird nachstehend anhand der Zeichnung beispielsweise näher erläutert. Es zeigen

Fig. 1 eine Ansicht im Schnitt eines Dreiwegverteilers zum Flüssikeitsaustausch,

Fig. 2 eine Ansicht des Dreiwegverteilers im Schnitt, wobei ein Nocken einer Schiebehülse in die äusserste Rasteröffnung der Steuernut eingreift,

Fig. 3 einen Schnitt entlang der Linie III-III der Fig. 2,

Fig. 4 eine Detailansicht im Schnitt der Kegelspitze des Anschlussschlauches, durch welche die Membran des Zuführungsschlauches durchgebrochen wurde,

Fig. 5 eine perspektivische Ansicht des Körpers des Dreiwegverteilers mit der Steuernut,

Fig. 6 eine Ansicht der abgewickelten Steuernut nach der Fig. 5 mit Rückzugverhinderungsmittel

Fig. 7 ein weiteres Ausführungsbeispiel eines Dreiwegverteilers im Schnitt,

Fig. 8 der Dreiwegverteiler nach Fig. 7, teilweise im Schnitt, wobei die Spitze eines rohrförmigen Kanals eine Membrane durchstossen hat,

Fig. 9 einen Schnitt durch eine andere Ausführungsform der Membrane, und

Fig. 10 eine schematische Ansicht eines Systems zum Ausführen der ambulanten peritonealen Heimdialyse.

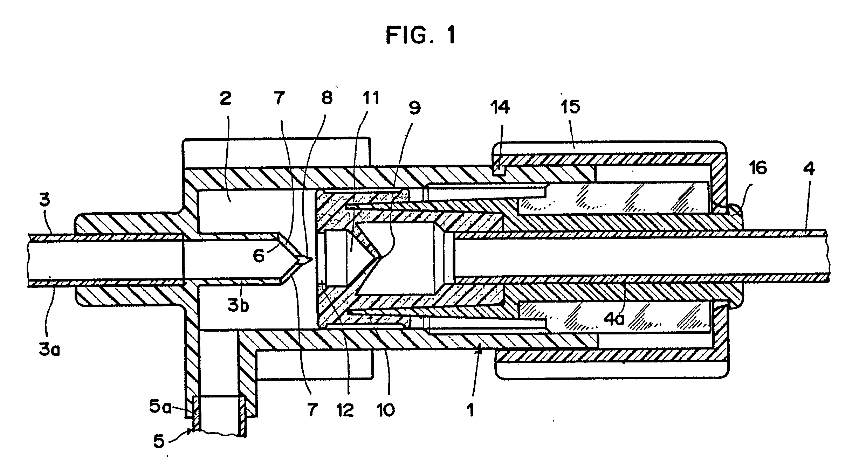

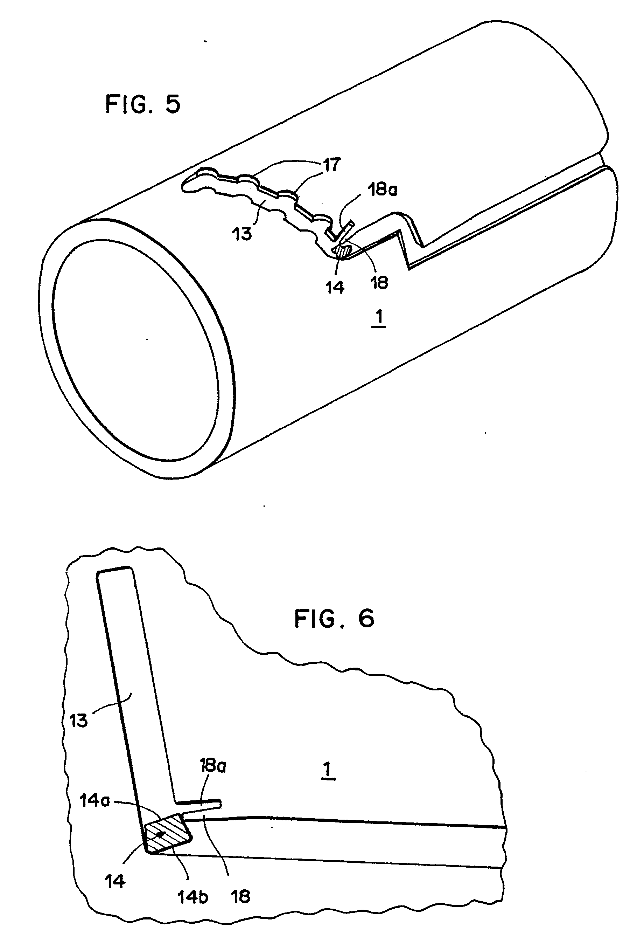

Der in den Figuren 1 bis 6 dargestellte Dreiwegverteiler dient zum Flüssigkeitsaustausch; er kann auf verschiedenen Gebieten, insbesondere dann in einem System zum Ausführen einer peritonealen Heimdialyse bei Patienten mit geschädigten Nieren, eingesetzt werden. Der Dreiwegverteiler besteht aus einem zylindrischen Körper 1 aus Kunststoffmaterial, in welchem eine Kammer 2 vorgesehen ist. Im Körper 1 des Dreiwegverteilers sind die einen Enden eines Anschlussschlauches 3, eines die frische Flüssigkeit zuführenden Zuführungsschlauches 4 und eines die verbrauchte Flüssigkeit abführenden Abführungsschlauches 5 dichtend eingesetzt.

Das eine Ende 4a des Zuführungsschlauches 4 ist mit dem einen Ende 3a des Anschlussschlauches 3 achsgleich ausgerichtet. Das eine Ende 5a des Abführungsschlauches 5 verläuft senkrecht zum einen Ende 3a des Anschlussschlauches 3 und mündet in der Kammer 2. Das eine Ende 3a des Anschlussschlauches 3 steht mit einem rohrförmigen Kanal 3b in Verbindung, der einen mit Oeffnungen 7 versehenen Kegel 6 mit Spitze 8 aus vorzugsweise hartem Kunststoffmaterial oder Metall aufweist. Das eine Ende 4a des Zuführungsschlauches 4, das gegenüber der Spitze 8 liegt, ist durch eine Membrane 9 abgeschlossen und mit der frischen Flüssigkeit gefüllt. Zum Zweck der Herstellung einer Durchflussverbindung zwischen dem Zuführungsschlauch 4 und dem Anschlussschlauch 3 über die Oeffnungen 7 des Kanals 3b ist das eine Ende 4a des Zuführungsschlauches 4 in der Kammer 2 axial verschiebbar angeordnet. Bei der Verschiebung dieses Endes 4a wird die Membrane 9 in Richtung des einen Endes 3a des Anschlussschlauches 3 bewegt und durch die Spitze 8 des Kanals 3b durchbrochen.

In der Aussenfläche des Körpers 1 des Dreiwegverteilers ist eine Steuernut 13 in Form einer Schraubenlinie oder eines Bajonettverschlusses eingearbeitet. Die Aussenfläche des Körpers 1 ist teilweise durch eine Schiebehülse 15 umschlossen. Die Schiebehülse 15 weist auf ihrem freien Ende einen Nocken 14 auf, der in die Steuernut 13 eingreift. Auf der anderen Seite steht die Schiebehülse 15 in Eingriff mit einem das eine Ende 4a des Zuführungsschlauches 4 dicht umgebenden Mantel 16.

Beim Verdrehen der Schiebehülse 15 stets in einer Richtung wird ihr Nocken 14 in verschiedenen Rasteröffnungen 17 der Steuernut 13 einrasten. Durch das Verdrehen der Schiebehülse 15 wird das eine Ende 4a des Zuführungsschlauches 4 gegen die Spitze 8 des Anschlussschlauches 3 gebracht, bis die Spitze 8 die Membrane 9 durchbricht. Dann kann die frische Flüssigkeit aus dem Zuführungsschlauch 4 durch die Oeffnungen 7 im Kegel 6 des Kanals 3b in den Anschlussschlauch 3 fliessen. Der Durchfluss der frischen Flüssigkeit wird bis zu einem maximalen Durchfluss abgestuft, indem der Nocken 14 stufenweise bis in die äusserste Rasteröffnung der Steuernut 13 gelangt. Auf diese Weise wird die Durchflussmenge der frischen Flüssigkeit gesteuert.

In der Abwicklung der Steuerkurve 13 ist ein den Rückzug des in die Steuernut 13 eingreifenden Nokkens 14 verhinderndes Mittel 18 eingebaut. Dieses als Sicherung dienende Mittel kann aus einer Feder mit Schlitz bestehen; es ist in der Steuernut 13 an der Stelle eingebaut, die der Stellung der Membrane 9 unmittelbar vor der Spitze 8 entspricht. Der in die Steuernut 13 eingeführte Vorderteil 14a des Nockens 14 kann aber auch so gestaltet sein, dass er gegenüber seinem Hinterteil 14b im Querschnitt verjüngt ist. Da die beiden parallelen Wände der Steuernut 13 (der Körper 1 besteht aus Kunststoffmaterial) elastisch nachgiebig sind, wird durch den breiteren Hinterteil 14b des Nockens 14 und durch die elastisch nachgiebigen parallelen Wände der Steuernut 13 der Rückzug des Nockens 14 verhindert.

Im unverschobenen Zustand des einen Endes 4a des Zuführungsschlauches 4 stehen die Oeffnungen 7 im Kegel 6 des an den Anschlussschlauch 3 angeschlossenen Kanals 3b in Verbindung mit der Kammer 2. In dieser Phase kann die über den Anschlussschlauch 3 abgeführte verbrauchte Flüssigkeit durch die Oeffnungen 7 in die Kammer 2 austreten und durch den Abführungsschlauch 5 abgeführt werden.

Das eine Ende 4a des Zuführungsschlauches 4 ist von einem im Querschnitt kreisförmigen Vorsprung 10 aus vorzugsweise weichem Kunststoffmaterial umschlossen. Der freie hervorragende Endteil dieses Vorsprunges 10 weist mittig eine der Form der Kegelspitze 6, 8 des Anschlussschlauches 3 entsprechende, durch die Membrane 9 abgeschlossene Aussparung 11 auf, die an ihrem Eintrittsrand mit einer Dichtlippe 12 versehen ist. Beim Verschieben des einen Endes 4a des Zuführungsschlauches 4 gegen die Kegelspitze 6, 8 des Anschlussschlauches 3 gelangt die Kegelspitze 6, 8 zuerst in die Aussparung 11, wonach die Membrane 9 durchbrochen wird. Diese Phase tritt dann ein, wenn der Nocken 14 in der äussersten Rasteröffnung 17 der Steuernut 13 einrastet. In dieser Phase ist die Kammer 2 des Körpers 1 durch den Vorsprung 10 vollständig dichtend ausgefüllt, so dass die dort möglicherweise vorher eingedrungene Luft in den Abführungsschlauch 5 verdrängt wird und die Einlauföffnung des Abführungsschlauches 5 in der Kammer 2 dichtend abgeschlossen wird.

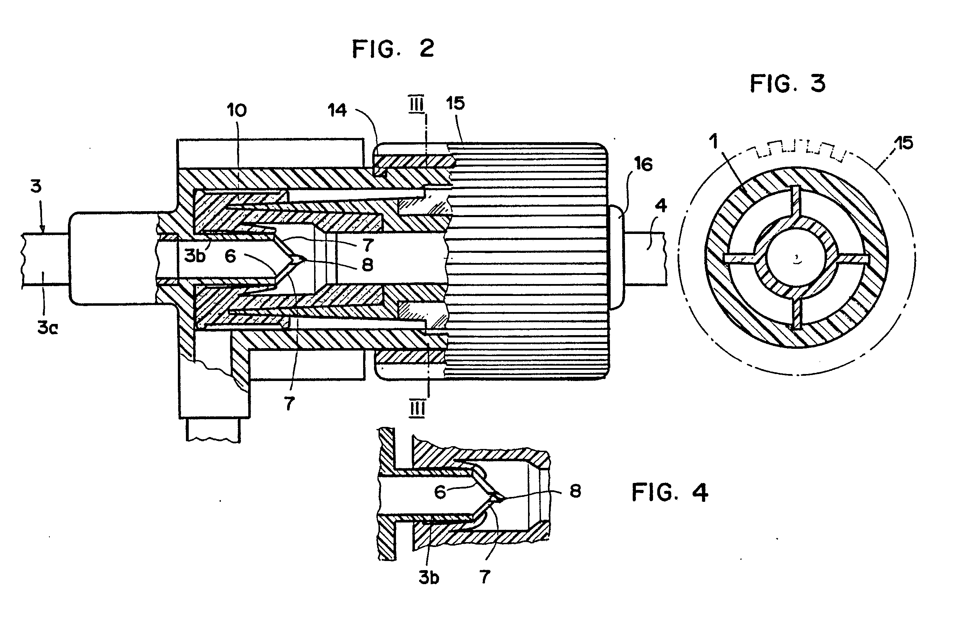

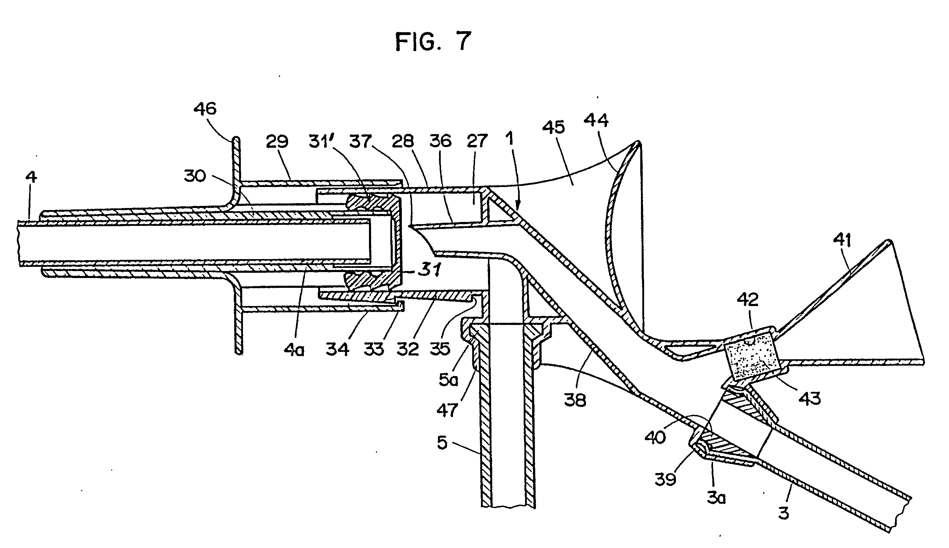

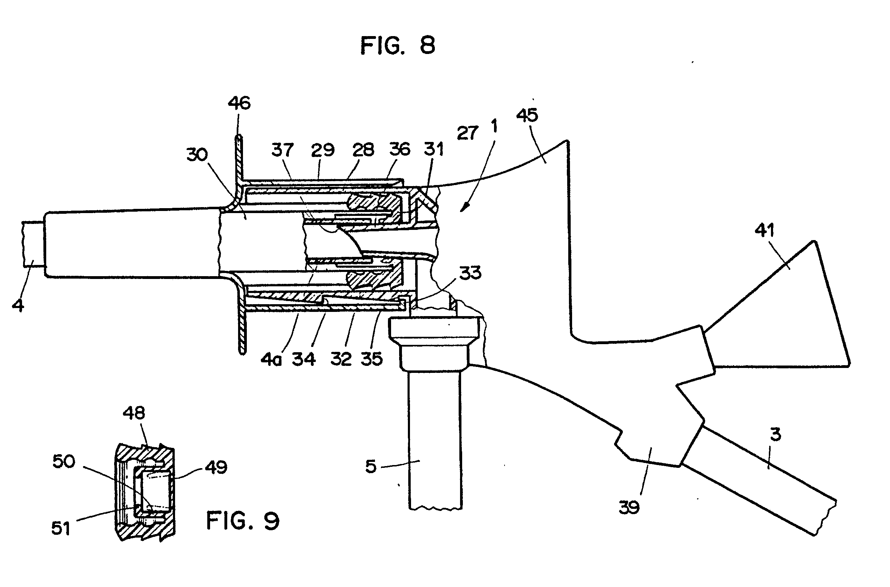

Die Fig. 7 und 8 zeigen ein weiteres Ausführungsbeispiel eines Dreiwegverteilers im Schnitt bzw. teilweise im Schnitt. Nur die völlig identischen Teile, wie der Anschlussschlauch 3, der Zuführungsschlauch 4 und der Abführungsschlauch 5, sowie die einen Enden 3a, 4a, 5a dieser Schläuche sind mit den gleichen Bezugszeichen wie beim Ausführungsbeispiel nach den Fig. 1 und 2 versehen.

Der eine Kammer 27 hauptsächlich begrenzende, teilweise zylindrische Körper 28 besteht vorzugsweise aus Kunststoff. Auf den zylindrischen Teil des Körpers 28 ist eine Schiebehülse 29 aufgesetzt, in die ein das eine Ende 4a dicht umschliessender Mantel 30 hineinragt. Das innere Ende des Mantels 30 ist durch eine Membran 31 verschlossen. Der verdickte zylindrische Randbereich 31' der Membrane 31 weist sowohl auf der Innen- als auch auf der Aussenseite ringförmige Rippen auf, die einerseits auf dem Mantel 30 und andererseits an der Innenseite des zylindrischen Teiles des Körpers 28 dichtend anliegen. Das vom Mantel 30 umgebene Ende 4a des Zuführungsschlauches 4 ist in den Mantel 30 eingeklebt und somit mit ihm fest verbunden. Am inneren Ende des Mantels 30 ist der Innendurchmesser desselben erweitert und ein Teil des Endes 4a des Zuführungsschlauches 4 ragt in den erweiterten Teil des Mantels 30 hinein.

An einer Stelle des zylindrischen Teiles des Körpers 28 ist eine sich achsial erstreckende, radial nach aussen vorstehende Rippe 32 angeordnet, welche im Querschnitt betrachtet sägezahnförmig ist. Im Bereich der Rippe 32 besitzt die Schiebehülse 29 einen radial nach innen vorstehenden Randbereich 33 zum Hintergreifen von sich radial erstreckenden Schultern 34 bzw. 35 der sägezahnförmigen Rippe 32. Die Schultern 34 und 35 verhindern, dass sich die Schiebehülse 29 bezogen auf die Fig. 7 und 8 nach links verschieben können, wenn der Randbereich 33 die eine oder die andere Schulter 34 bzw. 35 hintergriffen hat.

In die Kammer 27 ragt ein koaxial zum Mantel 30 der Schiebehülse 29 angeordneter, einen Teil eines rohrförmigen Kanals bildender Rohransatz 36. Das in die Kammer 27 mündende Ende des Rohransatzes 36 ist abgeschrägt und bildet eine Spitze 37 zum Durchstossen der Membrane 31, wenn die Schiebehülse 29 von der in der Fig. 7 gezeichneten Stellung in die in dem Fig. 8 dargestellte Stellung verbracht wird.

Der Rohransatz 36 ist über ein bezüglich der Längsachse der Schiebehülse 29 schräg angeordnetes Rohrstück 38 mit einem Verbindungsstück 39 verbunden. In das Verbindungsstück 39 ist das Ende 3a des Anschlussschlauches 3 mit einer konisch ausgebildeten Muffe 40 dichtend eingeklemmt. Unmittelbar neben dem Verbindungsstück 39 mündet ein Trichter 41 über einen Durchgang 42 in das Rohrstück 38. Im Durchgang zwischen dem Rohrstück 38 und dem Trichter 41 ist ein Pfropfen 43 aus einem emkstkwkjen Material angeordnet. Der Pfropfen 43 kann mittels einer Hohlnadel einer nicht dargestellten Spritze zum Injizieren eines Medikamentes in das Rohrstück 38 durchstossen werden. Der Trichter 41 dient zum Erleichtern des Einführens der Spritze. Nach dem Entfernen der Hohlnadel aus dem Pfropfen 43 verschliesst sich dieser wieder selbsttätig.

Vom mittleren Bereich des Rohrstückes 38 erstreckt sich eine als Auflage für den Daumen dienende gewölbte Wand 44, die über eine Stützwand 45 mit dem restlichen Teil des Rohrstückes 38 verbunden ist. Die Schiebehülse 29 weist einen radial nach aussen abstehenden Flansch 46 auf, der als Auflage für den Zeige- und den Mittel-finger der den Dreiwegverteiler bedienenden Person dient. Indem der Daumen an die gewölbte Wand 44 und die genannten Finger an den Flansch 46 angelegt werden, kann die Schiebehülse 29 bequem von der in der Fig. 7 gezeigten Stellung in die in der Fig. 8 gezeigte Stellung verbracht werden.

Die Kammer 27 steht über einen Anschlussstutzen 47 mit dem einen Ende 5a des Abführungsschlauches 5 in Verbindung. Der verdickte Randbereich des Endes 5a wird durch den Anschlussstutzen 47 formschlüssig festgehalten.

Die Schiebehülse 29, der Rohransatz 36, das Rohrstück 38, das Verbindungsstück 39, der Trichter 41 und die Wand 44 sind vorzugsweise aus einem Kunststoff hergestellt und sind vorerst als zwei Halbschalen gefertigt, wobei die Schnittebene der Fig. 7 und 8 die Trennebene der beiden Halbschalen ist. Nach dem Einsetzen des mit der Muffe 40 versehenen Endes 3a des Anschlussschlauches 3 in das geteilte Verbindungsstück 39, des Pfropfens 43 in den geteilten Durchgang zwischen dem Rohrstück 38 und dem Trichter 41 sowie des mit dem verbreiterten Randbereich versehenen Endes 5a des Abführungsschlauches 5 in den geteilten Anschlussstutzen 47 wird die andere Halbschale auf die erstere aufgesetzt und längs der Trennebene mit dieser verschweisst. Danach wird die Schiebehülse 29 samt des in deren Mantel 30 eingeklebten Endes 4a des Zuführungsschlauches 4 über den zylindrischen Teil des Körpers 28 aufgeschoben, bis der Randbereich 33 die erste Schulter 34 der Rippe 32 hintergreift.

Der Aussendurchmesser des in die Kammer 27 ragenden Endes des Rohransatzes 36 ist angenähert gleich dem Innendurchmesser des Endes 4a des Zuführschlauches 4. In Richtung zum Rohrstück 38 nimmt der Aussendurchmesser des Rohransatzes 36 leicht zu, so dass in der in der Fig. 8 gezeigten Stellung der Schiebehülse 29 das Ende 4a des elastischen Zuführungsschlauches 4 etwas aufgeweitet wird. Dies wird durch den erweiterten Innendurchmesser des Mantels 30 an seinem inneren Ende möglich und bewirkt eine absolut dichte verschlossene Verbindung zwischen dem Ende 4a und dem Rohransatz 36. Gleichzeitig ist die Verbindung zwischen dem Abführungsschlauch 5 und den beiden anderen Schläuchen 3 und 4 vollständig unterbunden.

Die Fig. 9 zeigt einen Schnitt durch eine bevorzugte Ausführungsform der in den Fig. 7 und 8 dargestellten Membrane 31. Der den Mantel 30 umfassende Teil 48 der modifizierten Membrane 49 weist ebenfalls radial nach aussen und innen vorstehende Rippen auf. Koaxial zum Teil 48 ist ein rohrförmiger Vorsprung 50 angeordnet, der an seinem freien Ende einen radial nach innen ragenden Wulst 51 aufweist. Diese modifizierte Membrane 49 wird verwendet, wenn nur ein kurzer Endbereich des Zuführungsschlauches 4 in den Mantel 30 hineinragt und der Innendurchmesser des Mantels 30 auf seiner ganzen Länge konstant ist. Das innere Ende des Mantels 30 befindet sich zwischen dem Teil 48 und dem rohrförmigen Vorsprung 50. Wenn der Rohransatz 36 die Membrane 49 durchstossen hat, liegt der Wulst 51 auf dem Rohransatz 36 an und bildet eine Dichtung, welche verhindert, dass aus der Kammer 27 Verunreinigungen in den Zuführungsschlauch 4 gelangen. Ueberdies können sich die Reste der durchstochenen Membrane 49 im ringförmigen Raum zwischen dem Rohransatz 36 und dem rohrförmigen Vorsprung 50 ansammeln.

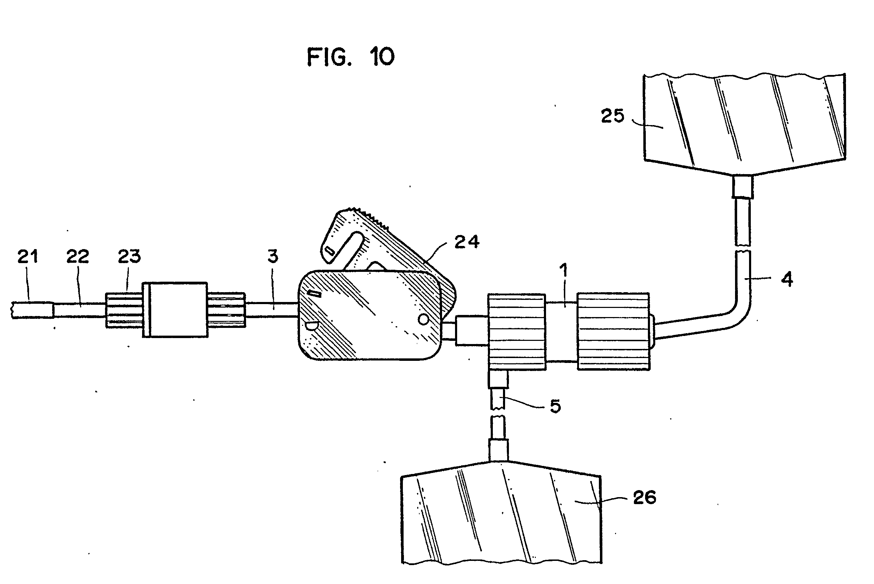

In der Fig. 10 ist ein System zum Ausführen einer peritonealen Heimdialyse bei Patienten mit geschädigten Nieren dargestellt, in welchem der Dreiwegverteiler nach der Fig. 1 eingesetzt ist. In dieser Figur ist mit 21 ein in der Bauchhöhle des Patienten eingelegter Katheter, mit 22 ein Verlängerungsstück des Katheters, mit 23 ein Verschluss, mit 3 der Anschlussschlauch, mit 24 ein Gerät zum Klemmen und Trennen des Anschlussschlauches 3, mit 1 der Körper des Dreiwegverteilers, mit 4 der Zuführungsschlauch der frischen Flüssigkeit aus einem Beutel 25 und mit 5 der Abführungsschlauch der verbrauchten Flüssigkeit in den Beutel 26 bezeichnet. Die von den geschädigten Nieren des Patienten ausgeschiedenen Stoffwechselprodukte werden aus der Bauchhöhle des Patienten durch das verbrauchte Dialysat abgeführt, wonach in die Bauchhöhle des Patienten frisches Dialysat eingeführt wird. Eine solche Dialyse kann der Patient selber, z.B. zuhause, durchführen.

Die oben beschriebenen Dreiwegverteiler stellen je eine komplett abgeschlossene bzw. abgedichtete Einheit mit klar definierten Durchflusswegen der Flüssigkeiten dar. Ihre Bedienung beschränkt sich auf die Verdrehung bzw. Verschiebung der Schiebehülse auf dem Körper des Dreiwegverteilers und das Einrasten des Nockens dieser Schiebehülse in den entsprechenden Rasteröffnungen der Steuernut. Der Durchfluss der Flüssigkeiten durch den Dreiwegverteiler erfolgt stets nur in einer Richtung, wobei die frische Flüssigkeit mit der verbrauchten Flüssigkeit nicht in Berührung kommt. Der aufwendige, lästige und immer eine gewisse Kontaminationsgefahr in sich bergende Durchspülungsvorgang, wie er aus der PCT-Anmeldung CH 84/0020 bekannt ist, fällt hier weg.

## Ansprüche

1. Dreiwegverteiler zum Flüssigkeitsaustausch mit einem Anschlussschlauch, einem die frische Flüssigkeit zuführenden Zuführungsschlauch und einem die verbrauchte Flüssigkeit abführenden Abführungsschlauch, wobei jeweils die einen Enden des Anschlussschlauches, des Zuführungsschlauches und des Abführungsschlauches in dem Dreiwegverteiler dichtend enden, dadurch gekennzeichnet, dass das eine Ende des Anschlussschlauches (3) in einen rohrförmigen Kanal (3b; 36) mündet, dass ein eine Kammer (2; 27) aufweisender Körper (1) vorhanden ist, in welchem das eine Ende (4a) des Zuführungsschlauches (4) und wenigstens der Endbereich des genannten Kanals (3b; 36) zueinander achsgleich ausgerichtet sind, dass das eine Ende (5a) des Abführungsschlauches (5) in die Kammer (2; 27) mündet, und dass das eine Ende (4a) des Zuführungsschlauches (4) abgeschlossen, mit der frischen Flüssigkeit gefüllt und zum Zweck der Herstellung einer Durchflussverbindung über den genannten Kanal (3b; 36) mit dem Anschlussschlauch (3) in der Kammer (2) achsial verschiebbar angeordnet ist.

2. Dreiwegverteiler nach Anspruch 1, dadurch gekennzeichnet, dass das eine Ende des Kanals (3b; 36) in einen mit Oeffnungen (7) versehenen Kegel (6) mit Spitze (8) aus hartem Material übergeht, oder zum Bilden einer Spitze (37) schräg angeschnitten ist und dass das eine Ende (4a) des Zuführungsschlauches (4) durch eine Membrane (9; 31) abgeschlossen ist.

3. Dreiwegverteiler nach Anspruch 2, dadurch gekennzeichnet, dass die Membrane (9; 31) bei der Verschiebung des einen durch sie abgeschlossenen Endes (4a) des Zuführungsschlauches (4) in Richtung des einen Endes des Kanals (3b; 36) durch die Spitze (8; 37) desselben durchbrechbar ist.

4. Dreiwegverteiler nach Anspruch 2 oder 3, dadurch gekennzeichnet, dass das eine Ende (4a) des Zuführungsschlauches (4) von einem Vorsprung (10) aus weichem Material umschlossen ist, dessen freier, hervorragender Endteil mittig eine der Form der Kegelspitze (6, 8) des Anschlussschlauches (3) entsprechende, durch die Membrane (9) abgeschlossene Aussparung (11) aufweist, die an ihrem Eintrittsrand mit einer Dichtlippe (12) versehen ist (Fig. 1).

5. Dreiwegverteiler nach Anspruch 1, dadurch gekennzeichnet, dass in der Aussenfläche seines Körpers (1) eine Steuernut (13) in Form einer Schraubenlinie oder eines Bajonettverschlusses eingearbeitet ist, in welche ein Nocken (14) einer die Aussenfläche des Körpers (1) teilweise umschliessenden Schiebehülse (15) eingreift, welche Schiebehülse mit einem das eine Ende (4a) des Zuführungsschlauches (4) dicht umgebenden Mantel (16) in Eingriff steht, dass die Durchflussmenge der frischen Flüssigkeit durch die Oeffnungen (7) in der Kegelspitze (6, 8) des Anschlussschlauches (3) durch das Einstellen des Nockens (14) in Rasteröffnungen (17) der Steuernut (13) steuerbar ist, und dass in der Steuernut (13) ein den Rückzug des in die Steuernut eingreifenden Nockens (14) verhinderndes Mittel (18) eingebaut ist (Fig. 5).

6. Dreiwegverteiler nach einem der Ansprüche 1 bis 3, dadurch gekennzeichnet, dass sich an den rohrförmigen Kanal (36) ein zur Längsrichtung desselben scnräg angeordnetes Rohrstück (38) anschliesst, dass an dem einen Ende des Rohrstückes (38) eine konisch ausgebildete Muffe (40) zum Einklemmen des einen Endes (3a) des Anschlussschlauches (3) vorhanden ist, dass ein Trichter (41) über einen Durchgang (42) mit dem Rohrstück (38) verbunden ist und dass der Durchgang (42) durch einen elastischen Pfropfen (43) verschlossen ist.

7. Dreiwegverteiler nach Anspruch 6, dadurch gekennzeichnet, dass die Kammer (27) teilweise durch einen zylindrischen Teil des Körpers (1) begrenzt ist, dass über dem zylindrischen Teil des Körpers (1) eine von einer ersten Stellung in eine zweite Stellung verschiebbare Schiebehülse (29) angeordnet ist, dass die Schiebehülse (29) grösstenteils einen Mantel (30) umgibt, und dass das eine Ende (4a) des Zuführungsschlauches (4) mittels Klebstoff in dem Mantel (30) befestigt ist.

8. Dreiwegverteiler nach Anspruch 7, dadurch gekennzeichnet, dass etwa im mittleren Bereich des Rohrstückes (38) eine als Anlage für den Daumen einer Bedienungsperson dienende Wand (44) angeordnet ist und dass die Schiebehülse (29) einen radial nach aussen abstehenden Flansch (46) zur Anlage des Zeige- und Mittelfingers der Bedienungsperson aufweist.

9. Dreiwegverteiler nach Anspruch 2, dadurch gekennzeichnet, dass die Oeffnungen (7) in der Kegelspitze (6, 8) im unverschobenen Zustand des einen Endes (4a) des Zuführungsschlauches (4) mit der Kammer (2) in Verbindung stehen.

10. Dreiwegverteiler nach Anspruch 1, dadurch gekennzeichnet, dass die Einlauföffnung des Abführungsschlauches (5) in der Kammer (2) bei vollständig verschobenem Zustand des einen Endes (4a) des Zuführungsschlauches (4) durch den Vorsprung (10) dichtend abgeschlossen ist.

11. Einrichtung zum Ausführen einer peritonealen Heimdialyse bei Patienten mit geschädigten Nieren mit einem Dreiwegverteiler nach Ansprüchen 1 bis 10.

## Claims

1. A three-way connector for the exchange of fluids, with a connection tube, a supply tube for supplying the fresh fluid and a drain tube for draining off the used fluid, one end of the connection tube, the supply tube and the drain tube ending fluid-tightly in the three-way connector, characterized in that one end of the connection tube (3) ends in a tubular duct (3b;

36), in that there is a body (1) having a chamber (2; 27), in which body one end (4a) of the supply tube (4) and at least the end region of said duct (3b; 36) are coaxially aligned, in that one end (5a) of the drain tube (5) ends in the chamber (2; 27), and in that one end (4a) of the supply tube (4) is closed, filled with the fresh fluid and is axially displaceable in the chamber (2) in order to produce a flow-through connection with the connection tube (3) via the said duct (3b; 36).

2. The three-way connector of claim 1, characterized in that one end of the duct (3b; 36) becomes a cone (6) provided with apertures (7) with a pointed tip (8) of a hard metal, or is bevelled to form a pointed tip (37) and in that one end (4a) of the supply tube (4) is closed by a diaphragm (9; 31).

3. The three-way connector of claim 2, characterized in that the diaphragm (9; 31) can be pierced by the pointed tip (8; 37) of the duct when one end (4a) of the supply tube (4) closed by the diaphragm is displaced in the direction of one end of the duct (3b; 36).

4. The three-way connector of claim 2 or 3, characterized in that one end (4a) of the supply tube (4) is enclosed by an appendage (10) of soft material whose free, projecting end portion has a central recess (11) of a shape which matches the cone tip (6, 8) of the connection tube (3), said recess being closed by the diaphragm (9) and having a sealing lip (12) on its rim (Fig. 1).

5. The three-way connector of claim 1, characterized in that into the outside surface of its body (1) is worked a control groove (13) in the form of a helical line or bayonet catch, with which groove a catch (14) of a sliding sleeve (15) partially surrounding the outside surface of the body (1) engages, said sliding sleeve being fixed to a jacket (16) which tightly surrounds one end (4a) of the supply tube (4), in that the rate of flow of fresh fluid through the openings (7) in the tip (6, 8) of the cone of the connection tube (3) can be controlled by adjusting the catch (14) in snap openings (17) of the groove (13), and in that means (18) to prevent a retraction of the catch (14) engaging in the groove are disposed in the groove (13) (Fig. 5).

6. The three-way connector of one of claims 1 to 3, characterized in that a tube piece (38) adjoins the tubular duct (36) and is disposed at an angle to the longitudinal direction thereof, in that on one end of the tube piece (38) there is a conical socket (40) to clamp one end (3a) of the connection tube (3), in that a funnel (41) is joined with the tube piece (38) via a passage portion (42) and in that the passage portion (42) is sealed by a resilient stopper (43).

7. The three-way connector of claim 6, characterized in that the chamber (27) is partially bounded by a cylindrical portion of the body (1), in that a sliding sleeve (29) which is displaceable from a first position to a second position is disposed over the cylindrical portion of the body (1), in that the sliding sleeve (29) substantially surrounds a jacket (30), and in that one end (4a) of the supply tube (4) is fixed in the jacket (30) by means of an adhesive.

8. The three-way connector of claim 7, characterized in that a wall (44) serving as thumb-rest for a user is disposed approximately in the central area of the tube piece (38) and in that the sliding sleeve (29) has an outwardly radially projecting flange (46) as a rest for the index and middle fingers of a user.

9. The three-way connector of claim 2, characterized in that in the non-displaced state of one end (4a) of the supply tube (4) the apertures (7) in the tip (6, 8) of the cone communicate with the chamber (2)

10. The three-way connector of claim 1, characterized in that in the fully displaced state of one end (4a) of the supply tube (4) the end of the drain tube (5) opening out in the chamber (2) is fluid-tightly closed by the appendage (10).

11. A device according to claims 1 to 10 with a three-way connector for carrying out peritoneal dialysis at home in patients with damaged kidneys.

**Revendications**

1. Valve à trois voies pour l'échange de liquides comprenant un tuyau de raccordement, un tuyau d'amenée amenant un liquide frais et un tuyau de vidage par lequel s'échappe le liquide usé, une extrémité de chacun des trois tuyaux de raccordement, d'amenée et de vidage aboutissant de manière étanche dans la valve à trois voies, caractérisée en ce que l'extrémité en question du tuyau de raccordement (3) débouche dans un canal tubulaire (3b; 36), en ce qu'un corps (1) comportant une chambre (2; 27) est présent, l'extrémité en question (4a) du tuyau d'amenée (4) et au moins la partie terminale dudit canal (3b; 36) étant disposées en correspondance axiale l'une avec l'autre dans ledit corps (1), en ce que l'extrémité en question (5a) du tuyau de vidage (5) débouche dans la chambre (2; 27), et en ce que l'extrémité en question (4a) du tuyau d'amenée (4), remplie du liquide frais, est obturée et disposée de façon axialement coulissante dans la chambre (2), dans le but d'établir une liaison de passage de flux avec le tuyau d'amenée (3) par ledit canal (3b; 36).

2. Valve à trois voies selon la revendication 1, caractérisée en ce qu'une des extrémités du canal (3b; 36) se restreint en un cône (6) ayant des ouvertures (7) et une pointe (8) d'un matériau dur, ou est taillée de façon oblique pour former une pointe (37), et en ce que l'extrémité en question (4a) du tuyau d'amenée (4) est fermée par une membrane (9; 31).

3. Valve à trois voies selon la revendication 2, caractérisée en ce que la membrane (9; 31) est perçable par la pointe (8; 37) de l'extrémité du canal (3b; 36) lors du déplacement en direction de l'extrémité du canal (3b; 36) de l'extrémité (4a) du tuyau d'amenée (4) qui est obturée par cette membrane (9; 31).

4. Valve à trois voies selon la revendication 2 ou 3, caractérisée en ce que l'extrémité en question (4a) du tuyau d'amenée (4) est entourée d'un manchon en projection vers l'avant (10), fait d'un matériau tendre, et dont l'extrémité libre, allant vers l'avant, présente, intérieurement, une forme correspondant à la pointe de cône (6, 8) du tuyau de raccordement (3), avec, au centre, une ouverture (11) obturée par la membrane (9), ce manchon (10) étant muni d'une lèvre d'étanchéité (12) à son bord d'entrée (fig. 1).

5. Valve à trois voies selon la revendication 1, caractérisée en ce que, dans la surface extérieure de son corps (1), est creusée une encoche de commande (13) sous la forme d'une ligne hélicoïdale ou d'une ligne de fermeture à baïonnette, encoche dans laquelle s'engage un ergot (14) d'une douille coulissante (15) qui entoure partiellement la surface extérieure du corps (1) et qui est en engagement avec un manteau (16) entourant de manière étanche l'extrémité en question (4a) du tuyau d'amenée (4), en ce que la quantité de liquide frais traversant l'ouverture (7) dont la pointe de cône (6, 8) du tuyau de raccordement (3) est commandable par la position de l'ergot (14) dans des creusures d'arrêtage (17) pratiquées dans l'encoche de commande (13), et en ce qu'un moyen (16) empêchant le retour de l'ergot (14) engagé dans l'encoche de commande (13) est monté dans cette encoche de commande (13) (fig. 5).

6. Valve à trois voies selon une des revendications 1 à 3, caractérisée en ce qu'une partie de tube (38), disposée obliquement par rapport à la direction longitudinale du canal tubulaire (36), se raccorde à ce dernier, en ce qu'un manchon (40), de configuration conique, est disposé a l'extrémité en question de cette partie de tube (38) pour enserrer l'extrémité en question (3a) du tuyau de raccordement (3), en ce qu'un entonnoir (41) est relié avec la partie de tube (38) par un passage (42), et en ce que ce passage (42) est fermé par un tampon élastique (43).

7. Valve à trois voies selon la revendication 6, caractérisée en ce que la chambre (27) est partiellement délimitée par une partie cylindrique du corps (1), en ce qu'une douille coulissante (29), pouvant être déplacée entre une première position et une deuxième position, est disposée sur la partie cylindrique du corps (1), en ce que cette douille coulissante (29) entoure en plus grande partie un manteau (30), et en ce que l'extrémité en question (4a) du tuyau d'amenée (4) est fixée dans ce manteau (30) au moyen d'un adhésif.

8. Valve à trois voies selon la revendication 7, caractérisée en ce que, approximativement, dans le domaine médian de la partie de tube (38) se trouve disposée une paroi (44) servant d'appui pour le pouce d'une personne utilisatrice, et en ce que la douille coulissante (29) présente un flanc (46) dressé radialement vers l'extérieur pour l'appui de l'index et du majeur de la personne utilisatrice.

9. Valve à trois voies selon la revendication 2, caractérisée en ce que les ouvertures (7) dans la pointe de cône (6, 8) se trouvent en liaison avec la chambre (2) dans la situation où l'extrémité en question (4a) du tuyau d'amenée (4) ne se trouve pas déplacée vers l'avant.

10. Valve à trois voies selon la revendication 1, caractérisée en ce que l'ouverture d'entrée du tuyau de vidage (5) est, dans la situation où l'extrémité en question (4a) du tuyau d'amenée (4) est complètement déplacé vers l'avant, isolée de manière étanche par le manchon en projection vers l'avant (10).

11. Installation pour l'exécution d'une dialyse péritonéale à domicile auprès de patients ayant les reins abîmés, utilisant une valve à trois voies selon les revendications 1 à 10.

FIG. 1

FIG. 2

FIG. 3

FIG. 4

FIG. 5

17

18a

13

14    18

1

FIG. 6

13

18a

14a

1

14

14b    18

FIG. 7

EP 0 250 369 B1

FIG. 8

FIG. 9

FIG. 10